# FASCICULE DE BREVET EUROPEEN

(11) **EP 1 171 003 B1**
(45) Date de publication et mention de la délivrance du brevet: **28.07.2004**
(21) Numéro de dépôt: 00922720.8
(22) Date de dépôt: 21.04.2000
(51) Int. Cl.: A23K 1/18

(54) **ALIMENT COMPLET POUR LARVES DE POISSONS ET PROCEDE POUR SA PREPARATION**
VOLLNAHRUNGSMITTEL FÜR FISCHLARVEN UND VERFAHREN ZU SEINER HERSTELLUNG
COMPLETE FEED FOR FISH LARVAE AND METHOD FOR PREPARING SAME

(30) Priorité: 21.04.1999 FR 9905049
(43) Date de publication de la demande: 16.01.2002
(73) Titulaire: INSTITUT NATIONAL DE LA RECHERCHE AGRONOMIQUE (INRA), 75341 Paris Cédéx 07 (FR); IFREMER INSTITUT FRANCAIS DE RECHERCHE POUR L'EXPLOITATION DE LA MER, 92130 Issy-les-Moulineaux (FR)
(72) Inventeur: ZAMBONINO, José, F-29280 Loc Maria Plouzane (FR); CAHU, Chantal, F-29200 Brest (FR); QUAZUGUEL, Patrick, F-29280 Loc Maria Plouzane (FR); BERGOT, Pierre, Rue Hiribehere, 64480 Ustaritz (FR)
(74) Mandataire: Catherine, Alain
(86) Numéro de dépôt international: PCT/FR2000/001068
(87) Numéro de publication internationale: WO 2000/064273

(56) Documents cités:
- EP-A- 0 292 052
- WO-A-00/27218
- WO-A-92/16115
- WO-A-97/42836
- GB-A- 1 318 463
- US-A- 5 661 981
- SARGEANT, J., MCEVOY, L., ESTEVEZ, A., BELL, G., BELL, M., HENDERSON, J., AND TOCHER, D.: "Lipid nutrition of marine fish during early development: current status and future directions" AQUACULTURE, vol. 179, septembre 1999 (1999-09), pages 217-229, XP000929740 AMSTERDAM, NL
- PATENT ABSTRACTS OF JAPAN vol. 007, no. 135 (C-170), 11 juin 1983 (1983-06-11) & JP 58 047447 A (RIKEN VITAMIN OIL KK), 19 mars 1983 (1983-03-19)
- PATENT ABSTRACTS OF JAPAN vol. 015, no. 123 (C-0816), 26 mars 1991 (1991-03-26) & JP 03 007546 A (NIPPON NOUSAN KOGYO KK;OTHERS: 08), 14 janvier 1991 (1991-01-14)
- CAHU, C., ZAMBONINO INFANTE, J., ESCAFFEE, A.-M., BERGOT, P., AND KAUSHIK, S.: "Preliminary results on sea bass (Dicentrarchus labrax) larvae rearing with compound diet from first feeding. Comparison with carp (Cyprinus carpio) larvae." AQUACULTURE, vol. 169, 1998, pages 1-7, XP000866313 cité dans la demande

## Description

La présente invention concerne une composition d'aliment complet pour larves de poissons, ainsi que son procédé de préparation.

Actuellement, la production d'alevins de poissons, et en particulier de poissons marins, comporte obligatoirement une phase d'alimentation par des proies vivantes, telles que les rotifères et les *Artemia.*

La durée d'utilisation des proies vivantes au cours d'un cycle de production d'alevins en écloserie varie suivant les espèces de poissons marins. Cette durée est généralement de 40 à 50 jours et peut atteindre une soixantaine de jours pour les espèces de poissons élevées en Europe.

L'utilisation d'un aliment de substitution aux proies vivantes, dit aliment de sevrage, n'intervient qu'ultérieurement, progressivement et généralement au-delà de quarante jours après l'éclosion.

L'utilisation de proies vivantes, et en particulier d'*Artemia,* dans l'alimentation des alevins au sein des écloseries présente de nombreux inconvénients.

La nécessité d'avoir recours à une structure d'élevage secondaire afin de produire les proies vivantes destinées à l'alimentation des alevins représente un premier inconvénient à la fois industriel et économique.

En second lieu, la qualité des proies vivantes et les possibilités d'approvisionnement en proies vivantes sont aléatoires et très variables selon les années. Il va sans dire que la qualité des proies vivantes a des conséquences directes sur le niveau de survie et de croissance des alevins alimentés avec ces dernières.

Il a ainsi été déterminé que l'utilisation d'un aliment de substitution aux proies vivantes pourrait présenter de nombreux avantages, en raison notamment d'une grande facilité de conservation et de stockage, d'une plus grande reproductibilité dans la qualité de l'apport alimentaire des alevins, et en conséquence une plus grande régularité des résultats quantitatifs et qualitatifs lors de l'élevage des larves de poissons.

Quelques tentatives de réalisation de compositions alimentaires utilisées comme substituts partiels ou totaux aux proies vivantes ont été décrites dans l'état de la technique.

La demande de brevet français publiée sous le n°2.572 625 décrit une composition alimentaire pour larves de poissons destinée à se substituer partiellement aux proies vivantes. Elle comprend de la poudre de jaune d'oeuf séché en mélange avec un lipide liquide à température ambiante.

D'autres substituts alimentaires sont décrits dans un premier article de CAHU et al. (1998, Aquaculture, vol. 169, pages 1-7), qui compare trois substituts alimentaires du point de vue de leur aptitude à assurer la survie et la croissance de deux types de poissons: le bar et la carpe.

Les résultats montrent que la composition de substitut présentant les meilleures propriétés quant au niveau de survie et de croissance des alevins possède un apport protéique comprenant, à parts égales, de la levure en poudre (Protibel) et un hydrolysat de protéines de poisson (respectivement 40% en poids de la matière sèche de la composition), de la lécithine de soja à raison de 5% en poids de la matière sèche, et d'un apport en lipides sous la forme de 2% en poids d'huile de poisson.

Selon les auteurs de cet article, bien que le substitut alimentaire en cause soit le meilleur des trois substituts testés, ces propriétés nutritives ne sont pas assez satisfaisantes pour envisager de l'utiliser en remplacement des proies vivantes.

Un autre article de CAHU et al. (1999, Aquaculture, vol. 171, pages 109-119) décrit quatre substituts alimentaires pour alevins, désignés respectivement HO, H19, H38 et H58.

Les auteurs proposent d'apporter une partie des protéines non seulement sous la forme de farine de poisson, mais aussi sous la forme d'une préparation d'hydrolysat de protéines de poisson à raison de 19%, 38,5% et 58% en poids de matière sèche respectivement des substituts H19, H38 et H58.

Ces substituts alimentaires ont été testés exclusivement sur des larves de bar qui ont été nourries à l'aide de l'une ou l'autre de ces quatre compositions dès le cinquième jour suivant l'ouverture de la bouche.(Jour 10 à compter de l'éclosion).

Les résultats expérimentaux montrent que la préparation H19 possède les qualités nutritives les meilleures, tant du point de vue du pourcentage de survie des larves que du point de vue de leur croissance et de leur qualité (taux de malformation osseuse et maturité des larves). La composition de substitut H19 comprend notamment, exprimé en pourcentage en poids de matière sèche, 58% de farine de poisson, 19% de concentré de protéine soluble de poisson, 5% de lécithine de soja, 4% d'huile de poisson, 8% d'un additif vitaminique ainsi que 5% d'un additif minéral.

Cet article a établi que la teneur optimale en concentré soluble de protéine de poisson, le seul paramètre variable des différentes compositions de substitut alimentaire testées dans cet article, était de 19%. Les auteurs ont conclu que l'incorporation de l'hydrolysat de protéines de poisson à cette proportion précise est capable d'accroître la qualité des larves, estimée par l'observation du taux de malformations du squelette.

On a maintenant trouvé selon l'invention, que la teneur en phospholipides de compositions destinées à l'alimentation de larves de poissons, en particulier de poissons marins, est une caractéristique essentielle au regard des qualités nutritives de telles compositions, et tout particulièrement en ce qui concerne leur capacité à maintenir un haut taux de survie chez les larves de poissons, à assurer à ces larves un fort taux de croissance, et à permettre l'obtention d'une population d'alevins d'excellente qualité, présentant un taux particulièrement bas de malformation squelettique et un haut degré de maturité physiologique.

L'invention a pour objet une composition selon la revendication 1.

Selon l'invention, il a été montré qu'un apport de phospholipides plus élevé que dans les compositions de l'état de la technique est essentiel pour réaliser un aliment complet donnant des taux de survie et de croissance des larves équivalents ou supérieurs à ceux obtenus avec des proies vivantes comme les rotifères et les *Artemia.* Les phospholipides sont présents dans le vitellus de l'oeuf qui est utilisé par l'embryon de poisson pour son développement jusqu'à ce qu'il soit capable d'ingérer des aliments exogènes. Ils sont aussi présents dans les proies vivantes. Les phospholipides doivent également être apportés en quantité suffisante dans les aliments complets pour les larves de poissons alors qu'un tel apport n'est pas obligatoire dans les aliments pour les poissons adultes.

La teneur en phospholipides de l'aliment complet suivant l'invention est comprise entre 8,5 et 25% en poids de la matière sèche de l'aliment, préférentiellement entre 10 et 20%.

Selon un aspect de l'invention, une teneur finale supérieure à 8,5% de phospholipides totaux dans l'aliment est garantie par l'addition d'au moins 6% de phospholipides d'origine végétale, qui s'additionnent aux phospholipides éventuellement apportés par les autres ingrédients.

On peut utiliser des lécithines d'origine végétale, comme la lécithine de soja, ou de la lécithine d'autres plantes oléagineuses telles que le tournesol ou le colza.

Il est précisé que le terme de lécithine désigne des mélanges lipidiques dans lesquels les phospholipides représentent plus de 50% des lipides totaux. Les classes de phospholipides les plus abondantes dans les lécithines sont la phosphatidylcholine, la phosphatidyléthanolamine et le phosphatidylinositol. Les lécithines commerciales peuvent se présenter avec une richesse variable en phospholipides, notamment sous forme déshuilée (environ 90% de phospholipides) ou non déshuilée (environ 50% de phospholipides). Ainsi, l'addition de 6% de phospholipides peut être réalisée aussi bien par l'addition de 7% de lécithine à 90% de phospholipides que par l'addition de 12% de lécithine à 50% de phospholipides.

Les lécithines végétales contiennent des acides gras en C18 des séries d'acides gras indispensables n-3 et n-6 (acide linoléique: 18:2n-6 et acide linolénique: 18:3n-3) mais sont dépourvues d'acides gras polyinsaturés en C20 et C22, notamment l'acide arachidonique (20:4n-6), l'acide eicosapentaénoïque (EPA, 20:5n-3) et docosahexaénoïque (DHA, 22:6n-3). Ceci ne limite pas l'utilisation des lécithines car ces derniers acides gras peuvent être apportés, indépendamment des lécithines ajoutées, par les autres ingrédients de l'aliment. Ce dernier apport peut se faire sous forme de triglycérides ou de phospholipides, en utilisant en particulier des produits provenant des poissons comme les huiles de poissons ou les farines de poissons. La composition pour aliment complet selon l'invention contient un apport de lipides neutres, provenant de la farine de poisson et contenant des acides gras longs polyinsaturés, notamment de la série (n-3). Les acides gras de la série (n-3) représentent 2,5% en poids de la matière sèche dans l'aliment selon l'invention. Compte-tenu de cet apport complémentaire possible en acides gras, la composition en acides gras des lécithines n'est pas critique et des lécithines modifiées comme des lécithines hydrogénées peuvent également être employées.

Selon un autre aspect de l'invention, la teneur en lipides totaux de l'aliment est supérieure à 25% en poids de la matière sèche. La présence d'une quantité suffisante de phospholipides dans l'aliment, outre son intérêt spécifique, améliore l'utilisation digestive des autres constituants lipidiques de l'aliment et notamment celle des lipides neutres et en particulier celle des triglycérides. Un des avantages d'accroître la teneur en lipides des aliments est d'augmenter leur contenu en énergie digestible pour la couverture des besoins énergétiques des larves.

Il a été démontré selon l'invention qu'une composition d'aliment contenant, suivant un aspect de l'invention, 17% de lécithine de soja non déshuilée (% de la matière sèche de l'aliment) et présentant une teneur en phospholipides totaux de 12% (correspondant à environ 9% provenant de la lécithine et 3% provenant des autres ingrédients de l'aliment) assurait des taux de survie et de croissance bien supérieurs à ceux qui étaient obtenus dans l'état de la technique avec le substitut H19 évoqué ci-avant.

En outre, on a démontré que les résultats de survie et de croissance obtenus avec un aliment suivant l'invention sont équivalents ou supérieurs à ceux qui étaient obtenus avec des proies vivantes, c'est-à-dire avec des rotifères durant les premiers jours d'alimentation et avec les Artemia dans les jours suivants.

Selon un autre aspect, la teneur en protéines de poissons non hydrolysées de la composition pour aliment complet selon l'invention est supérieure à 50% en poids de la matière sèche.

L'apport en protéines non hydrolysées est de préférence constitué de farine de poisson qui entre dans la composition pour aliment complet à raison de 45 à 60% en poids de la matière sèche et est de manière tout à fait préférée d'environ 56% en poids de la matière sèche.

Selon un autre aspect de l'invention, la composition pour aliment complet comprend en outre une teneur en protéines hydrolysées, de préférence sous la forme d'un concentré protéique soluble de poisson, comprise entre 8 et 25%, de préférence entre 10 et 22% et de manière tout à fait préférée d'environ 14% en poids de la matière sèche de la composition.

Avantageusement, une composition pour la préparation d'un aliment complet pour larves de poissons selon l'invention comprendra en outre un additif vitaminique.

On peut par exemple mettre en oeuvre un mélange vitaminique dont la composition en vitamines, exprimée par kilo de mélange vitaminique est la suivante:
acétate de rétinol (1g);
cholécalciferol (2,5 mg);
acétate de all-rac-α-tocophérol (10g);
ménadione (1 g);
thiamine (1 g);
riboflavine (0,4 g);
pantothénate de D-calcium (2g);
pyridoxine HCl (0,3 g);
cyanocobalamine (1g);
niacine (1 g);
chlorure de choline (200g);
acide ascorbique (20g);
acide folique (0,1 g) ;
biotine (1g) ; et
méso-inositol (30 g).

La composition d'aliment complet pour larves de poissons selon l'invention peut en outre comprendre un additif minéral.

De préférence, un tel additif minéral est constitué d'un mélange minéral de constitution suivante, exprimé par kilogramme de mélange minéral:

KCI (90g); Kl (40 mg); CaHPO₄.2H₂O (500 g); NaCl (40 g); CuSO₄ 5H₂O (3g); ZnSO₄.7H₂O (4g); CoSO₄.7H₂O (20 mg); FeSO₄.7H₂O (20g); MnSO₄.H₂O (3g); CaCO₃ (215g); MgSO₄.7H₂O (124g) et NaF (1g).

L'apport en vitamines et en composés minéraux de la composition pour aliment complet selon l'invention peut être qualitativement et quantitativement adapté en fonction des teneurs finales en vitamines et minéraux résultant de l'apport des autres matières premières utilisées dans la préparation de cette composition, comme par exemple la teneur en vitamines et minéraux des sources en protéines, lipides et phospholipides.

Avantageusement, la composition pour aliment complet selon l'invention peut encore comprendre de faibles quantités de facteurs d'appétence tels que la bétaïne.

De manière préférée, de tels facteurs d'appétence seront utilisés dans les compositions selon l'invention à raison de 0,5 à 3% en poids de la matière sèche, par exemple à raison d'environ 1% en poids de matière sèche.

En outre, la teneur en eau finale de la composition d'aliment complet pour larves de poissons selon l'invention n'est pas supérieure à 14% en poids de la composition, et est de préférence comprise entre 7 et 10% en poids et est de manière tout à fait préférée d'environ 8% en poids de la composition finale.

De manière tout à fait préférée, une composition pour aliment complet selon l'invention aura la composition suivante, exprimée en poids de matière sèche :

**TABLEAU I**

| Ingrédients | % de matière sèche |
|---|---|
| Farine de poisson | 56 |
| Concentré protéique soluble de poisson | 14 |
| Lécithine de soja | 17 |
| Mélange vitaminique | 8 |
| Mélange minéral | 4 |
| Bétaïne | 1 |

Selon un autre aspect de l'invention, une telle composition aura la constitution suivante, exprimée en poids de matière sèche:

| | |
|---|---|
| Protéines totales (N x 6,25) | 56% |
| Phospholipides totaux | 12% |
| Lipides totaux | 26% |
| Cendres | 14% |

La composition d'aliment complet pour larves de poissons selon l'invention peut être avantageusement utilisée pour préparer un aliment composé complet pour l'alimentation de larves de poissons présentées sous la forme de granulés de granulométrie définie.

De préférence, la taille des granulés d'aliment composé complet est telle que la plus grande dimension est inférieure à 600µm. La taille des granulés peut être ajustée pour préparer un aliment composé complet convenant à des larves de poissons d'âges différents ou encore à des larves de poissons d'espèces ayant des tailles différentes.

Ainsi, selon un premier mode de réalisation de l'aliment composé complet pour larves de poissons selon l'invention, la taille des granulés est comprise entre 400 et 600 µm.

Selon un second aspect de l'aliment composé complet selon l'invention, la taille des granulés est comprise entre 200 et 400 µm.

Selon un troisième aspect de l'aliment composé complet pour larves de poissons, la taille des granulés est comprise entre 120 et 200µm.

Enfin, selon un quatrième aspect de l'aliment composé complet pour larves de poissons selon l'invention, la taille des granulés est inférieure à 120µm.

L'aliment composé complet pour larves de poissons décrit ci-dessus, possède des qualités nutritives qui le destine à un usage étendu, c'est-à-dire à la fois pour nourrir des poissons d'eau douce et des poissons marins.

De préférence, l'aliment composé complet pour larves de poissons selon l'invention est destiné à l'alimentation des poissons d'eau de mer quelle qu'en soit l'origine. Il est en effet non seulement adapté à l'alimentation des poissons européens tels que les bars, les daurades ou encore le turbot, mais aussi à l'alimentation des poissons exotiques d'élevage tels que le lates ou encore l'ombrine.

L'aliment composé complet pour larves de poissons selon l'invention a des qualités nutritives telles qu'il permet un taux de survie ainsi qu'un taux de croissance des larves encore jamais atteints ainsi que la production de larves ayant un taux de malformations morphologiques particulièrement bas et un degré de maturité exceptionnel, comparé aux substituts alimentaires décrits dans l'état de la technique.

L'invention a en outre pour objet un procédé pour la préparation d'un aliment composé complet pour larves de poissons, ledit procédé étant caractérisé en ce qu'il comporte les étapes suivantes:
a) obtention d'une pâte par mélange d'eau et d'au moins trois matières premières dans les proportions suivantes:
   (i) farine de poisson en proportion de 50 à 65 % en poids du mélange final;
   ii) concentré protéique soluble de poisson en proportion de 8 à 25% en poids du mélange final;
   iii) lécithine de soja en proportions de 7 à 25% en poids du mélange final;
b) granulation par voie humide de la pâte ainsi obtenue sous forme de filaments ;
   c) congélation des filaments de pâte à basse température, de préférence à une température inférieure à -80°C ;
   d) concassage et/ou broyage des filaments de pâte congelée à l'étape c) ;
   e) tamisage des granulés obtenus lors de l'étape de concassage.

L'étape de broyage ou de concassage des filaments extrudés est particulièrement facilitée du fait de l'étape de congélation préalable. En effet, la pâte initiale résultant du mélange de matières premières additionnées d'eau possède une concentration importante en lipides, ce qui a pour effet de colmater les broyeurs et les tamis utilisés pour fabriquer les particules d'aliments.

Au contraire, la congélation préalable permet de présenter la composition initiale sous une forme solide et friable particulièrement adaptée aux étapes de concassage puis tamisage.

Le procédé selon l'invention est en outre caractérisé en ce que de l'eau est ajoutée au moment du mélange initial de l'étape a), à raison de 10 à 15% du poids du mélange. Cette étape particulière a pour fonction de permettre le collage des particules des différents constituants de matière première pour l'obtention d'une pâte homogène à l'issue de l'étape initiale de mélange.

Le procédé selon l'invention comprend en outre avantageusement une étape de séchage des filaments de pâte obtenus après l'étape b) de granulation afin d'ajuster la teneur en eau de la composition des filaments à une valeur inférieure à 14%, de préférence à une valeur comprise entre 7 et 10% et de manière tout à fait préférée à une valeur d'environ 8%.

L'étape de séchage peut être avantageusement réalisée à la température de 50°C pendant 20 minutes.

L'étape de séchage précède l'étape de congélation; elle est préférentiellement réalisée à la suite de l'étape b) de granulation par voie humide.

L'invention concerne encore un procédé d'élevage de larves de poissons, caractérisé en ce que les larves sont alimentées à raison de 1 à 4g par jour et par 2500 larves, avec l'aliment complet ci-dessus décrit.

L'invention sera en outre illustrée par les figures et les exemples suivants.

### DESCRIPTION DETAILLEE DES FIGURES

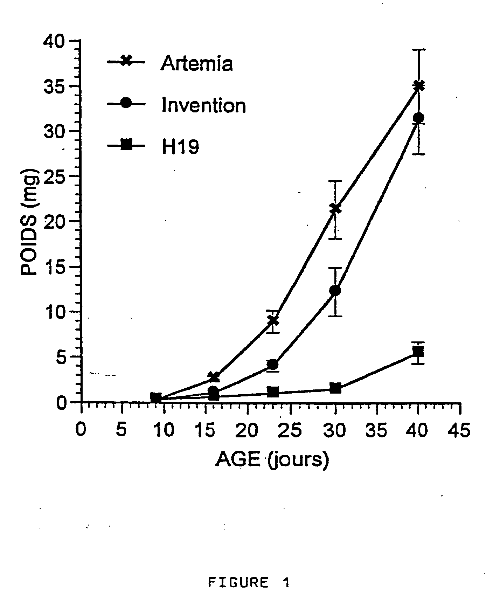

### Figure 1: Croissance des larves de bar nourries par des proies vivantes ou par des substituts alimentaires.

En ordonnées: poids des larves exprimé en mg de matière humide ;

En abscisse : âge des larves exprimé en nombre de jours après l'éclosion.

La croissance a été suivie du jour 9 au jour 40 après l'éclosion pour des larves nourries du jour 9 au jour 40 exclusivement de proies vivantes (*Artemia*), d'une composition pour aliment complet selon l'invention ou de la composition H19 de l'état de la technique (CAHU et al., 1999). Chaque point représente le résultat de la moyenne ± écart type de 4 lots de 10 larves.

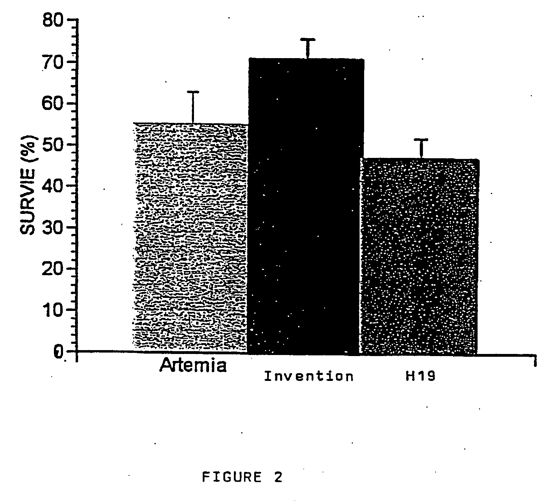

### Figure 2: Etude de la survie des larves au jour 40.

En ordonnées: taux de survie des larves exprimé en pourcentages de larves vivantes calculés sous forme du rapport de larves vivantes au jour 40 au nombre de larves vivantes au jour 2 après l'éclosion.

Le pourcentage de survie a été déterminé pour des larves nourries exclusivement par des proies vivantes (*Artemia*), un substitut alimentaire de l'état de la technique H19 et la composition selon l'invention pour aliment complet selon l'invention. Chaque point représente le résultat de la moyenne ± écart type de 4 lots.

### EXEMPLES

### EXEMPLE 1: Préparation d'un aliment composé complet pour poissons selon l'invention.

Dans un premier temps, les matières premières utilisées, et notamment la farine de poisson, sont tamisées avant leur mélange, de telle manière à obtenir des particules de chacune des matières premières ayant une taille inférieure à 160 µm.

Les matières premières sont les suivantes :
- la farine de poisson est la farine commercialisée sous la dénomination NORSE™ LT94, de teneur en protéines supérieure à 78% en poids de la matière sèche, et commercialisée par la Société NORSILDMEL.
- le concentré protéique soluble de poisson (hydrolysat de protéines de poisson) est le CPSP™ spécial G, commercialisé par la Société SOPROPECHE.
- la lécithine de soja utilisée, désignée sous la référence D10, est commercialisée par la Société SAPA-DAFA.
- la bétaïne se présente sous la forme chlorhydrate d'une pureté supérieure à 99% et est commercialisée sous la référence B3501 par la Société Sigma.
- la mélange vitaminique utitisé est commercialisé sous la référence 762 par la Société UPAE-INRA.
- le mélange minéral utilisé est commercialisé sous la référence 763 par la Société UPAE-INRA.

Les matières premières ci-dessus ont été mélangées dans un bol en acier mû par un bras mécanique, après addition de 10% d'eau (en poids du mélange initial) afin d'assurer le collage des particules des differents constituants.

Le mélange homogène des matières premières passe ensuite à travers une filière afin de former de longs filaments cylindriques (« spaghetti ») de 3 mm de diamètre.

Les filaments de matière première sont ensuite séchés afin de ramener la teneur en eau à environ 8% en poids du mélange final. Le séchage est réalisé dans une étuve à flux d'air chaud à la température de 50°C pendant 20 minutes.

Les filaments d'un diamètre de 3 mm dont la teneur en eau a été ajustée sont ensuite soumis à une étape de congélation à l'azote liquide pendant une durée d'une dizaine de secondes.

Les filaments congelés sont ensuite soumis à une étape de concassage dans un broyeur et mis sous forme de granulés par des moyens connus. De préférence, une basse température est maintenue durant cette étape, de manière à éviter un colmatage du broyeur.

Les granulés obtenus à l'étape précédente sont ensuite congelés à l'azote liquide et passés sur des tamis. Des tamis successifs ont été utilisés de telle manière à séparer les granulés en différentes fractions de taille respectivement comprise entre 400 et 600 µm, 200 et 400µm, 120 et 200µm et enfin de taille inférieure à 120 µm.

Les granulés ainsi obtenus ont une teneur en eau de 8% de la matière sèche. La constitution précise de la composition pour aliment complet est la suivante :

| Ingrédients | % de matière sèche |
|---|---|
| Farine de poisson | 56 |
| Concentré protéique soluble de poisson | 14 |
| Lécithine de soja | 17 |
| Mélange vitaminique | 8 |
| Mélange minéral | 4 |
| Bétaïne | 1 |

La composition des granulés, exprimée par rapport à la matière sèche de l'aliment est de :
- protéines totales (N x 6,25): 56%;
- phospholipides totaux: 12%;
- lipides totaux: 26%;
- cendres : 14%.

### EXEMPLE 2 : SURVIE ET CROISSANCE DES LARVES DE BAR NOURRIES AVEC L'ALIMENT COMPOSE COMPLET SELON L'INVENTION ET COMPARAISON AVEC UN SUBSTITUT ALIMENTAIRE REPRESENTATIF DE L'ETAT DE LA TECHNIQUE POUR DES LARVES AGEES DE 9 A 40 JOURS.

Des larves de bar (*Dicentrarchus labrax*) ont été réparties dans des bacs de 35 litres à une densité de 60 larves/l. L'ensemble des larves a été alimenté par des rotifères (*Brachionus plicatilis*) durant une période de temps de 3 jours suivant l'ouverture de la bouche

Les larves ont ensuite été séparées en deux groupes de quatre lots et la survie et la croissance des larves ont été suivies du jour 9 au jour 40 après l'éclosion (c'est-à-dire du jour 4 au jour 35 après l'ouverture de la bouche).

Le premier groupe de quatre lots a été nourri exclusivement de *nauplii d'Artemia.* Le second groupe de quatre lots a été exclusivement nourri avec l'aliment composé complet selon l'invention conforme à l'exemple 1.

Les autres conditions d'élevage ont été uniformisées dans les bassins, la température de l'eau étant maintenue à 20°C avec une salinité de 35 ppm. Les bassins ont été éclairés en permanence.

L'alimentation des larves est réalisée à l'aide de distributeurs automatiques de type tapis roulant fixés au-dessus des bassins et l'aliment a été fourni aux larves en continu (24/24).

Les quantités journalières d'aliment composé complet ont été de 1 g par jour pour 2500 larves en début du cycle de nourrissage et ont été progressivement augmentées jusqu'à 4 g/j pour 2500 larves en fin de cycle de nourrissage (J 40 après l'éclosion).

Les quantités d'aliments utilisées correspondent à une distribution en excès permettant une disponibilité permanente des granulés ou des proies vivantes pour les larves.

Selon le procédé de distribution alimentaire retenu, l'aliment composé complet décante lentement dans la colonne, ce qui rend possible la capture des granulés par les larves au cours de cette décantation lente.

Chaque jour, les dépôts d'aliments excédentaires et de fèces accumulés au fond des bassins sont enlevés sans remise en suspension.

Après 32 jours d'alimentation (40 jours après l'éclosion), les résultats, exprimés comme la moyenne ± écart-type de la moyenne, sont les suivants:

| | Aliment complet | Artémia | H19 |
|---|---|---|---|
| Survie (%) | 70 ± 6,9 | 55 ± 8,1 | 47 ± 6,2 |
| Poids moyen (mg) | 32± 3,8 | 35 ± 4,1 | 6 ± 0,8 |

Les résultats obtenus dans les mêmes conditions d'élevage avec le substitut alimentaire H19 représentatif de l'état de la technique (CAHU et al., 1999) sont également présentés à titre de comparaison.

La survie indiquée correspond au nombre de larves en fin d'expérience (40ème jour après l'éclosion) exprimé en pourcentage du nombre initial de larves mises en élevage deux jours après l'éclosion (Figure 2).

Le poids moyen individuel a été calculé à partir de quatre prélèvements de 10 larves pour chacun des bassins d'un même lot expérimental.

La croissance des larves a été suivie du jour 9 au jour 40 après l'éclosion et les résultats expérimentaux sont représentés sur la figure 1.

Les résultats de la figure 1 montrent que les effets de l'aliment composé complet selon l'invention sur la croissance des larves sont tout à fait comparables, voire identiques, aux propriétés des *nauplii d'Artemia.*

Au jour 40 après l'éclosion, les larves nourries avec l'aliment composé complet selon l'invention ont un poids (32 mg) qui ne diffère pas significativement du poids des larves nourries avec les *Artemia* (35 mg ).

En revanche, les larves nourries à l'aide du substitut alimentaire H19 de l'état de la technique ont un taux de croissance extrêmement faible, puisque le poids des larves nourries avec ce substitut au jour 40 est d'environ 5 mg, soit un poids 6 fois moindre que celui obtenu avec l'aliment composé complet selon l'invention.

60 jours après l'éclosion, les larves n'ayant jamais reçu *d'Artemia* pesaient 300 mg en moyenne et présentaient un développement harmonieux, notamment au niveau du squelette.

Les résultats du tableau indiquent une survie significativement plus importante chez les larves ayant reçu l'aliment composé complet selon l'invention que les larves nourries avec les Artemia ou le substitut alimentaire H19.

Ces résultats démontrent clairement que l'aliment composé complet selon l'invention remplace avantageusement l'*Artemia* dans la séquence d'élevage des larves de bar et que ce substitut alimentaire permet même un taux de survie des larves significativement supérieur au taux de survie obtenu par une alimentation des larves à l'aide de proies vivantes.

### EXEMPLE 3: SURVIE ET CROISSANCE DES LARVES DE BAR NOURRIES AVEC L'ALIMENT COMPOSE COMPLET SELON L'INVENTION POUR LES LARVES AGEES DE 5 A 10 JOURS.

Les conditions générales de l'élevage des larves de bar sont celles exposées dans l'exemple 2. A l'ouverture de la bouche, soit 5 jours après l'éclosion, les larves ont été séparées en 2 groupes, le premier groupe de 4 lots a été nourri exclusivement de rotifères, le second groupe de 4 lots a été nourri avec l'aliment composé complet selon l'invention. Les particules de l'aliment avaient une taille inférieure à 120 µm.

Après 5 jours d'alimentation, les poids moyens étaient de 0,85 ± 0,07 mg pour les larves nourries avec l'aliment composé selon l'invention alors qu'il n'atteignait que 0,76 ± 0,10 mg pour les larves nourries avec les rotifères. La survie était identique dans les deux groupes (92%).

Cet exemple montre que l'aliment complet selon l'invention remplace avantageusement les rotifères et peut être utilisé comme aliment unique pendant tout l'élevage des larves de poisson.

Cet aliment présente un intérêt pour des espèces dont l'élevage des larves fait intervenir une longue utilisation de rotifères, tel que le turbot ou la dorade.

### EXEMPLE 4 : ETUDE COMPARATIVE DE LA QUALITE ET DE LA MATURITE DES LARVES DE BAR NOURRIES AVEC L'ALIMENT COMPOSE COMPLET SELON L'INVENTION OU LE SUBSTITUT ALIMENTAIRE H19.

Des paramètres physiologiques ont été mesurés dans les larves provenant de l'expérience décrite dans l'exemple 2.

Au 40ème jour de développement, seulement 3,8 ± 1,5% des larves nourries avec l'aliment composé complet selon l'invention présentaient une malformation squelettique. Ce taux était de 6 ± 1,2 % chez les larves nourries avec le substitut alimentaire désigné H19 dans l'article de CAHU et al. 1999.

L'état de maturité des fonctions digestives a été évalué par deux paramètres: la sécrétion d'une enzyme pancréatique et l'apparition dans les bordures en brosses des entérocytes d'une enzyme caractéristique de la digestion de type adulte, la phosphatase alcaline.

Les larves nourries avec l'aliment selon l'invention présentaient un taux de sécrétion de trypsine de 55 ± 5,5%, supérieur à celui des larves nourries avec le substitut alimentaire désigné H19, 43 ± 4,9%.

L'activité de la phosphatase alcaline atteignait 308 ± 10,7 mU/mg de protéines chez les larves nourries avec l'aliment suivant invention et était de 110 ± 8,9 mU/mg pour les larves nourries avec le substitut alimentaire H19.

L'utilisation de l'aliment suivant l'invention est avantageux pour produire des larves de bonne qualité morphologique et physiologique. Notamment, la maturation des fonctions digestives au cours de la vie larvaire est indispensable pour un bon développement ultérieur de poisson.

## Revendications

1. Composition d'aliment complet pour larves de poissons, **caractérisée en ce qu'**elle possède une teneur en phospholipides comprise entre 10 et 20 % en poids de la matière sèche dudit aliment et une teneur en protéines totales comprise entre 40 et 60 % en poids de la matière sèche dudit aliment.

2. Composition selon la revendication 2, **caractérisée en ce qu'**elle possède une teneur en phospholipides d'origine végétale au moins égale à 6% de la matière sèche de l'aliment.

3. Composition selon l'une des revendications 1 et 2, **caractérisée en ce qu'**elle possède une teneur en lipides totaux supérieure à 25% en poids de la matière sèche.

4. Composition selon l'une des revendications 1 à 3, **caractérisée en ce qu'**elle comporte une teneur en protéines totales égale à 50% en poids de la matière sèche.

5. Composition selon l'une des revendications 1 à 4, **caractérisée en ce qu'**elle possède une teneur en protéines hydrolysées comprise entre 8 et 25% en poids de la matière sèche, plus particulièrement comprise entre 10 et 22%, et de préférence égale à environ 14% en poids de la matière sèche.

6. Composition selon l'une des revendications 1 à 5, **caractérisée en ce qu'**elle comprend en outre un mélange additif vitaminique.

7. Composition selon l'une des revendications 1 à 6, **caractérisée en ce qu'**elle comporte en outre un mélange additif minéral.

8. Composition selon l'une des revendications 1 à 7, **caractérisée en ce qu'**elle comprend en outre un facteur d'appétence, tel que la bétaïne.

9. Composition selon l'une quelconque des revendications 1 à 8, **caractérisée en ce qu'**elle possède une teneur en eau inférieure à 14% en poids de la composition finale.

10. Composition selon l'une des revendications 1 à 9, **caractérisée par** la constitution suivante:
| Ingrédients | % en poids de matière sèche |
|---|---|
| Farine de poisson | 56 |
| Concentré protéique soluble de poisson | 14 |
| Lécithine de soja | 17 |
| Mélange vitaminique | 8 |
| Mélange minéral | 4 |
| Bétaïne | 1 |

11. Aliment composé complet pour larves de poissons constitué de granulée d'une composition selon l'une des revendications 1 à 10.

12. Aliment selon la revendication 11, **caractérisé en ce que** la taille des granulés est inférieure à 600µm, les différentes fractions de taille étant 600-400 µm, 400-200µm, 200-120µm et inférieure à 120 µm.

13. Procédé pour la préparation d'un aliment complet selon l'une des revendicatlons 11 et 12, **caractérisé en ce qu'**il comporte les étapes suivantes :
a) obtention d'une pâte par mélange d'eau et d'au moins trois matières premières dans les proportions suivantes :
(i) farine de poisson en proportion de 50 à 65% en poids du mélange final ;
(ii) concentré protéique soluble de poisson en proportion de 8 à 25% en poids du mélange final ;
(iii) lécithine de soja en proportion de 7 à 25% en poids du mélange final ;
b) granulation par voie humide de la pâte ainsi obtenue sous forme de filaments ;
c) congélation des filaments de pâte à basse température, de préférence à une température inférieure à -80°C ;
d) concassage et/ou broyage des filaments de pâte congelée à l'étape c) ;
e) tamisage des granulés obtenus lors de l'étape de concassage.

14. Procédé selon la revendication 13, **caractérisé en ce que**, lors de l'étape a) de mélange, de l'eau est ajoutée à raison de 10 à 15% du poids du mélange.

15. Procédé selon l'une des revendications 13 et 14, **caractérisé en ce que** l'étape b) de granulation est suivie d'une étape de séchage afin d'ajuster la teneur en eau des filaments à une valeur inférieure à 14%, de préférence à une valeur comprise entre 7 et 10% et de manière tout à fait préférée à une valeur de 8% environ.

16. Procédé d'élevage de larves de poissons, **caractérisé en ce que** les larves sont alimentées à raison de 1 à 4g, par jour et par 2500 larves, avec un aliment complet selon l'une des revendications 11 et 12.

## Patentansprüche

1. Vollnahrungszusammensetzung für Fischlarven, **dadurch gekennzeichnet, dass** sie einen Gehalt an Phospholipiden zwischen 10 und 20 Gew.-% der Trockenmasse des Futters und einen Gehalt an Gesamtproteinen zwischen 40 und 60 Gew.-% der Trockenmasse des Futters besitzt.

2. Zusammensetzung gemäß Anspruch 1, **dadurch gekennzeichnet, dass** sie einen Gehalt an Phospholipiden pflanzlichen Ursprungs von wenigstens 6 Gew.-% der Trockenmasse des Futters besitzt.

3. Zusammensetzung gemäß einem der Ansprüche 1 und 2, **dadurch gekennzeichnet, dass** sie einen Gehalt an Gesamtlipiden von über 25 Gew.-% der Trockenmasse besitzt.

4. Zusammensetzung gemäß einem der Ansprüche 1 bis 3, **dadurch gekennzeichnet, dass** sie einen Gehalt an Gesamtproteinen von 50 Gew.-% der Trockenmasse besitzt.

5. Zusammensetzung gemäß einem der Ansprüche 1 bis 4, **dadurch gekennzeichnet, dass** sie einen Gehalt an hydrolysierten Proteinen zwischen 8 und 25 Gew.-% der Trockenmasse, insbesondere zwischen 10 und 22 Gew.-% und vorzugsweise von ungefähr 14 Gew.-% der Trockenmasse besitzt.

6. Zusammensetzung gemäß einem der Ansprüche 1 bis 5, **dadurch gekennzeichnet, dass** sie außerdem ein Vitaminzusatzgemisch umfasst.

7. Zusammensetzung gemäß einem der Ansprüche 1 bis 6, **dadurch gekennzeichnet, dass** sie außerdem ein Mineralzusatzgemisch umfasst.

8. Zusammensetzung gemäß einem der Ansprüche 1 bis 7, **dadurch gekennzeichnet, dass** sie außerdem einen Appetenzfaktor, wie Betain, umfasst.

9. Zusammensetzung gemäß einem der Ansprüche 1 bis 8, **dadurch gekennzeichnet, dass** sie einen Wassergehalt von unter 14 Gew.-% der endgültigen Zusammensetzung besitzt.

10. Zusammensetzung gemäß einem der Ansprüche 1 bis 9, **gekennzeichnet durch** die folgende Zusammensetzung:
| Bestandteile | Gew.-% der Trockenmasse |
|---|---|
| Fischmehl | 56 |
| lösliches Fischproteinkonzentrat | 14 |
| Sojalecithin | 17 |
| Vitamingemisch | 8 |
| Mineralgemisch | 4 |
| Betain | 1 |

11. Vollnahrungsmischung für Fischlarven, die aus Körnern mit einer Zusammensetzung gemäß einem der Ansprüche 1 bis 10 besteht.

12. Nahrung gemäß Anspruch 11, **dadurch gekennzeichnet, dass** die Größe der Körner kleiner als 600 µm ist, wobei die verschiedenen Größenfraktionen 600-400 µm, 400-200 µm, 200-120 µm und kleiner als 120 µm sind.

13. Verfahren zur Herstellung einer Vollnahrung gemäß einem der Ansprüche 11 und 12, **dadurch gekennzeichnet, dass** es die folgenden Schritte umfasst:
a) Gewinnung einer Paste durch Mischen von Wasser und wenigstens drei Rohstoffen in den folgenden Anteilen:
(i) Fischmehl in einem Anteil von 50 bis 65 Gew.-% der Endmischung;
(ii) lösliches Fischproteinkonzentrat in einem Anteil von 8 bis 25 Gew.-% der Endmischung;
(iii) Sojalecithin in einem Anteil von 7 bis 25 Gew.-% der Endmischung;
b) feuchtes Granulieren der so erhaltenen Paste in Form von Filamenten;
c) Gefrierenlassen der Pastenfilamente bei tiefer Temperatur, vorzugsweise bei einer Temperatur von unter -80 °C;
d) Zerkleinern und/oder Mahlen der in Schritt c) gefrorenen Pastenfilamente;
e) Sieben des im Zerkleinerungsschritt erhaltenen Granulats.

14. Verfahren gemäß Anspruch 13, **dadurch gekennzeichnet, dass** das Wasser im Schritt des Mischens a) in einer Menge von 10 bis 15 Gew.-% des Gemischs hinzugefügt wird.

15. Verfahren gemäß einem der Ansprüche 13 und 14, **dadurch gekennzeichnet, dass** auf den Granulierungsschritt b) ein Trockenschritt folgt, um den Wassergehalt der Filamente auf einen Wert von unter 14%, vorzugsweise auf einen Wert zwischen 7 und 10% und ganz besonders bevorzugt auf einen Wert von ungefähr 8% einzustellen.

16. Verfahren zur Aufzucht von Fischlarven, **dadurch gekennzeichnet, dass** die Larven in einer Menge von 1 bis 4 g pro Tag und pro 2500 Larven mit einer Vollnahrung gemäß einem der Ansprüche 11 und 12 gefüttert werden.

## Claims

1. A complete feed composition for fish larvae, **characterized in that** it has a phospholipid content between 10 and 20% by weight of the dry material of said feed and a whole protein content between 40% and 60% by weight of the dry material of said feed.

2. A composition according of claim 1, **characterized in that** it has a phospholipid content from vegetal origin at least equal to 6% of the feed dry material.

3. A composition according to one of claims 1 and 2, **characterized in that** it has a whole lipid content higher than 25% by weight of the dry material.

4. A composition according to one of claims 1 to 3, **characterized in that** it has a whole protein content equal to 50% by weight of the dry material.

5. A composition according to one of claims 1 to 4, **characterized in that** it has a hydrolyzed protein content ranging from 8 to 25% by weight of the dry material, more particularly ranging from 10 to 22%, and preferably equal to about 14% by weight of the dry material.

6. A composition according to one of claims 1 to 5, **characterized in that** it comprises moreover a vitamin additive blend.

7. A composition according to one of claims 1 to 6, **characterized in that** it comprises moreover a mineral additive blend.

8. A composition according to one of claims 1 to 7, **characterized in that** it comprises moreover an appetizing factor, such as betaine.

9. A composition according to any one of claims 1 to 8, **characterized in that** it comprises a water content less than 14% by weight of the final composition.

10. A composition according to one of claims 1 to 9, **characterized in that** it has the following formulation :
| Ingredients | % of dry material |
|---|---|
| Fish meal | 56 |
| Fish soluble protein concentrate | 14 |
| Soya lecithin | 17 |
| Vitamin blend | 8 |
| Mineral blend | 4 |
| Betaine | 1 |

11. A complete mixed feed for fish larvae made of granules having a composition according to one of claims 1 to 10.

12. A feed according to claim 11, **characterized in that** the granule size is less than 600 µm, the different size fractions ranging from 600 to 400 µm, 400 to 200 µm, 200 to 120 µm and less than 120 µm.

13. A method for preparing a complete feed according to one of claims 11 and 12, **characterized in that** it comprises the following steps of :
a) obtaining a paste by mixing water and at least three raw materials in the following proportions :
(i) fish meal in a quantity ranging from 50 to 65% by weight of the final blend;
(ii) fish soluble protein concentrate in a quantity ranging from 8 to 25% by weight of the final blend;
(iii) soya lecithin in a quantity ranging from 7 to 25% by weight of the final blend;
b) granulating, by wet route, the paste thus obtained in the form of filaments;
c) deep-freezing the paste filaments at a low temperature, preferably at a temperature less than -80°C;
d) crushing and/or grinding the deep-frozen paste filaments, from step c);
e) sieving the granules obtained in the crushing step.

14. A method according to claim 13, **characterized in that** water is added, upon the blending step a), in an amount ranging from 10 to 15% by weight of the blend.

15. A method according to one of claims 13 and 14, **characterized in that** the granulating step b) is followed by a drying step in order to adjust the filament water content to a value less than 14%, preferably to a value ranging from 7 to 10% and most preferably to a value equal to about 8%.

16. A fish larvae breeding method, **characterized in that** the larvae are fed in an amount ranging from 1 to 4g/day and per 2500 larvae, with a complete feed according to one of claims 11 and 12.
